# FASCICULE DE BREVET EUROPEEN

(11) **EP 3 622 992 B1**
(45) Date de publication et mention de la délivrance du brevet: **27.01.2021**
(21) Numéro de dépôt: 19194948.6
(22) Date de dépôt: 02.09.2019
(51) Int. Cl.: A61M 16/00, G06F 3/0484, G06F 3/0488

(54) **SYSTÈME DE CONTRÔLE MANUEL D'UN APPAREIL D'ASSISTANCE À LA TOUX**
MANUELLES KONTROLLSYSTEM EINES GERÄTS ZUR HILFE BEI HUSTEN
MANUAL CONTROL SYSTEM FOR A DEVICE FOR ASSISTING WITH COUGHS

(30) Priorité: 14.09.2018 FR 1871042
(43) Date de publication de la demande: 18.03.2020
(73) Titulaire: EOVE, 64000 Pau (FR)
(72) Inventeur: COTTEAUX, Fabien, 64000 PAU (FR); CORNET, Allan, 64000 PAU (FR)
(74) Mandataire: Air Liquide

(56) Documents cités:
- WO-A1-2008/102216
- WO-A1-2011/139194
- WO-A1-2016/159889
- WO-A1-2017/006189
- WO-A2-2012/158394
- WO-A2-2014/184377
- US-A1- 2010 020 021
- US-A1- 2016 202 890

## Description

La présente invention concerne un appareil médical d'insufflation et d'exsufflation de gaz, aussi appelé appareil d'assistance à la toux, utilisable pour traiter une personne, i.e. un patient, souffrant de troubles respiratoires nécessitant une aide à l'évacuation des sécrétions pulmonaires, avec système de contrôle manuel par commande tactile.

Les dispositifs ou appareils d'assistance à la toux sont des appareils de thérapie respiratoire. Ces appareils comprennent généralement une turbine motorisée, aussi appelée « compresseur » ou « micro-soufflante », tournant à vitesse donnée, et un circuit de gaz comprenant une branche d'insufflation pour amener le gaz au patient pendant les phases d'insufflation et une branche d'exsufflation pour convoyer le gaz extrait du patient, pendant les phases d'exsufflation, avant son rejet à l'atmosphère. La branche d'insufflation et la branche d'exsufflation (internes à l'appareil) sont généralement reliées fluidiquement à une branche commune les reliant au patient, via un conduit flexible et une interface respiratoire, tel que masque ou autre.

La turbine d'un appareil d'assistance à la toux est habituellement couplée à une ou plusieurs électrovannes ou analogue permettant d'orienter alternativement le flux d'air :
- pendant les phases d'insufflation : depuis la turbine vers le patient, c'est-à-dire avec sortie d'air sous pression de la turbine connectée fluidiquement au patient, via la branche d'insufflation et la branche commune, et
- pendant les phases exsufflation : depuis le patient vers la turbine, c'est-à-dire avec patient raccordé fluidiquement à l'entrée d'air de la turbine, via la branche commune et la branche d'exsufflation, de sorte d'opérer une aspiration et obtenir une pression négative dans la branche commune et la branche d'exsufflation.

EP-A-2707069 enseigne un exemple d'un tel appareil d'assistance à la toux.

Habituellement, des capteurs de pression et de débit associés à un contrôleur compris dans des moyens de pilotage permettent de gérer les différentes phases en fonction des réglages choisis.

Dans un dispositif d'assistance à la toux, le gaz chemine donc alternativement depuis la sortie de la turbine vers le patient en phase d'insufflation et depuis le patient vers l'entrée de la turbine en phase exsufflation.

Dans certains cas, le fonctionnement de ce type de dispositif d'assistance à la toux, en particulier les phases d'insufflation et d'exsufflation, doit être commandé manuellement.

Pour ce faire, on prévoit généralement un système de contrôle manuel qui est intégré au dispositif, par exemple un interrupteur à bascule mécanique, actionnable manuellement par l'utilisateur entre une position d'insufflation et une position d'exsufflation. Chaque pivotement de l'interrupteur à bascule enclenche une phase d'insufflation ou d'exsufflation en fonction de la position dans laquelle il est actionné. Il existe aussi en général une position intermédiaire ou médiane, dans laquelle aucune phase n'est déclenchée, c'est-à-dire que dans cette position médiane, l'interrupteur est au repos ou le traitement du patient en pause.

Cependant les systèmes de contrôle actuels des appareils d'assistance à la toux posent certains problèmes ou inconvénients, notamment :
- ils sont souvent encombrants puisqu'entièrement mécaniques,
- ils ne sont pas agréables à utiliser et peuvent même blesser l'utilisateur lorsque la séance de thérapie dure plusieurs dizaines de minutes ou plus, puisque celui-ci doit actionner l'interrupteur à bascule mécanique alternativement entre les positions d'insufflation et d'exsufflation pendant toute cette durée,
- ils fonctionnent avec une certaine inertie puisqu'un certain temps de latence est obligatoirement nécessaire pour basculer d'une position à une autre, et/ou
- ils imposent un passage, même bref, en position médiane, c'est-à-dire en mode « pause », qui peut dégrader la performance globale du traitement.

Dans d'autres appareils, tels ceux décrits par WO-A-2012/158394 et WO-A-2016/159889, le contrôle des phases d'insufflation et d'exsufflation se fait au moyen de deux boutons ou touches dédiés devant être activés alternativement et répétitivement par un utilisateur, c'est-à-dire qu'il doit d'abord appuyer sur la touche d'insufflation puis sur celle d'exsufflation, puis répéter ces actions autant de fois que nécessaire.

On comprend que ce type de contrôle à deux boutons ou touches n'est pas idéal car pouvoir respecter un rythme régulier entre les activations successives des boutons ou touches servant à déclencher les phases d'insufflation et d'exsufflation peut être difficile pour un utilisateur.

De plus, il requiert une grande attention de sa part car celui-ci doit appuyer correctement sur les boutons ou touches pour que l'action soit prise en compte par l'appareil, à défaut de quoi la phase désirée n'est pas déclenchée par l'appareil, par exemple si l'utilisateur appuie mal, pas assez fort ou à côté de la touche à activer. D'ailleurs, si l'utilisateur est un médecin ou un autre personnel soignant, celui-ci doit être attentif au patient pour suivre son rythme, donc ne peut pas être concentré sur les boutons à activer. Utiliser deux boutons ou touches représente donc une contrainte d'utilisation importante.

Enfin et surtout, avec ce type d'appareils à deux boutons ou touches, entre le relâchement du premier bouton et l'appui sur le second bouton, la transition n'est pas immédiate puisqu'il existe un temps de latence, comme avec les dispositifs à bascule mécanique susmentionnés. Ce temps de latence engendre des performances en expiration, c'est-à-dire pendant les phases exsufflatoires, de l'appareil qui ne sont pas assez efficaces pour le patient, alors que c'est justement que l'on recherche à obtenir avec ce type d'appareil.

Le problème est donc de proposer un appareil d'assistance à la toux amélioré comprenant un système de contrôle manuel permettant à un utilisateur de déclencher manuellement les phases d'insufflation et d'exsufflation, lequel système de contrôle manuel ne présente pas tout ou partie des problèmes ou inconvénients susmentionnés, en particulier sans le temps de latence des appareils à deux boutons.

La solution de l'invention concerne alors un appareil d'insufflation et d'exsufflation de gaz à un patient, c'est-à-dire destiné au traitement d'un patient souffrant de troubles respiratoires nécessitant une aide à l'évacuation des sécrétions pulmonaires, aussi appelé appareil d'assistance à la toux, comprenant un afficheur comprenant une zone tactile, et des moyens de pilotage commandant une turbine motorisée, les moyens de pilotage étant configurés pour commander la turbine en réponse à la détection d'au moins une pression digitale d'un utilisateur sur la zone tactile de l'afficheur, caractérisé en ce que les moyens de pilotage sont configurés pour :
a) opérer un démarrage de la turbine et déclencher une phase d'insufflation de la turbine en réponse à la détection d'un déplacement, i.e. un glissement, du doigt de l'utilisateur sur, i.e. au contact de, ladite zone tactile selon un premier sens de déplacement, et
b) déclencher une phase d'exsufflation de la turbine en réponse à la détection d'un déplacement du doigt de l'utilisateur sur ladite zone tactile selon un deuxième sens de déplacement, le premier sens de déplacement et le deuxième sens de déplacement étant des sens opposés.

La solution de l'invention permet d'obtenir un appareil à toux dans lequel le passage d'une phase d'insufflation à une phase d'exsufflation est instantané grâce à la détection des glissements du doigt de l'utilisateur en « va-et-vient » sur la zone tactile de l'appareil, typiquement sur un écran tactile, comme détaillé ci-après.

En effet, tout arrêt du balayage digital avec maintien du contact du doigt de l'utilisateur avec la zone tactile, continue l'action du balayage venant d'être effectué dans une première direction de balayage, donc la phase d'insufflation ou d'exsufflation en cours.

Dès lors, dès que l'utilisateur débute un balayage digital dans le sens opposé, i.e. une deuxième direction de balayage orientée en sens opposé à la première direction de balayage, le changement d'état est instantané détecté par l'appareil, c'est-à-dire sans aucune latence, et l'autre phase d'exsufflation ou d'insufflation, respectivement, est alors débutée.

Ceci améliore considérablement la performance de l'appareil, en particulier sa réactivité, donc forcément aussi les soins apportés au patient.

Dans le cadre de l'invention :
- par « pression digitale », on entend un contact du doigt d'un utilisateur avec la surface de la zone tactile de l'afficheur, c'est-à-dire le fait que l'utilisateur touche la surface de cette zone tactile avec son doigt, i.e. contact digital, typiquement avec l'index. Dans le cadre de l'invention, le contact digital est immédiatement suivi d'un déplacement/glissement du doigt sur la surface de la zone tactile de l'afficheur.
- par « déplacement du doigt » de l'utilisateur sur la zone tactile, on entend que l'utilisateur opère un mouvement, glissement ou balayage de son doigt en le maintenant en permanence, au contact de la surface de la zone tactile, typiquement sur une distance d'un à quelques centimètres, i.e. < 10 cm environ.
- le terme « sens » est considéré comme équivalent au terme « direction ».

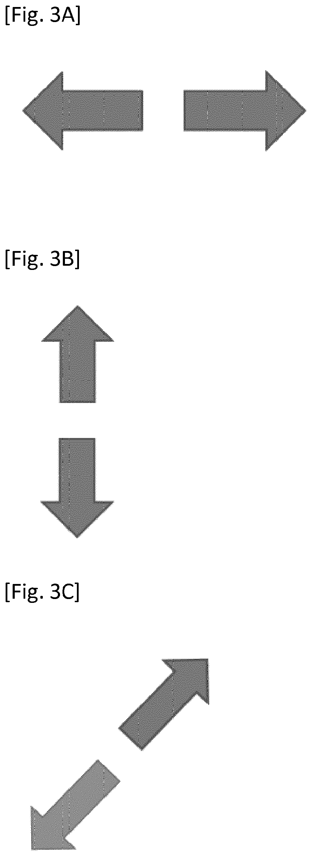

Selon le cas, l'appareil de l'invention peut comprendre l'une ou plusieurs des caractéristiques techniques suivantes :
- la zone tactile de l'afficheur comprend un pavé tactile ou « *touch pad* » en anglais.
- la zone tactile s'étend sur tout ou partie de la surface de l'afficheur, c'est-à-dire sur une zone localisée de l'écran d'affichage ou sur tout l'écran d'affichage.
- la zone tactile s'étend préférentiellement sur toute la surface tactile de l'afficheur.
- l'afficheur est un écran à affichage numérique, i.e. affichage digital, typiquement un écran tactile.
- les moyens de pilotage sont configurés pour opérer une phase d'insufflation de la turbine comprenant une fourniture de gaz par ladite turbine, typiquement de l'air.
- le premier sens de déplacement et le deuxième sens de déplacement sont des sens opposés ou approximativement opposés.
- le premier sens de déplacement et le deuxième sens de déplacement sont des sens prédéfinis ou non, de préférence prédéfinis, en particulier des sens conventionnels, et préférentiellement mémorisés, i.e. préenregistrés.
- le premier sens de déplacement et le deuxième sens de déplacement sont des sens prédéfinis modifiables ou non-modifiables.
- selon un premier mode de réalisation, le premier sens de déplacement et le deuxième sens de déplacement sont des sens prédéfinis. Par exemple, le premier sens de déplacement est celui de la gauche vers la droite (i.e. sens gauche/droite) et déclenche une phase d'insufflation, et le deuxième sens de déplacement est celui de la droite vers la gauche (i.e. sens droite/gauche) et déclenche une phase d'exsufflation.
- selon un deuxième mode de réalisation, le premier sens de déplacement et le deuxième sens de déplacement sont des sens prédéfinis modifiables, c'est-à-dire pouvant être changés, en particulier pouvant être inversés, notamment via un menu de configuration s'affichant sur l'afficheur.
- selon un troisième mode de réalisation, le premier sens de déplacement correspond à la direction du premier déplacement du doigt de l'utilisateur sur la zone tactile. Dans ce cas, l'appareil est configuré pour déclencher une phase d'insufflation de gaz, lors du premier glissement du doigt de l'utilisateur sur la zone tactile, c'est-à-dire que le sens de déplacement du doigt de l'utilisateur lors du premier contact et glissement de doigt sur la zone tactile définit le sens de l'insufflation de gaz (i.e. le premier sens de déplacement). Le sens opposé est alors celui de l'exsufflation (i.e. deuxième sens de déplacement en direction opposée à celle du premier sens de déplacement). Les moyens de pilotage sont configurés pour mémoriser le sens du premier glissement du doigt de l'utilisateur sur la zone tactile en tant que premier sens de déplacement, ce qui implique que le sens opposé est alors préférentiellement mémorisé comme étant celui de l'exsufflation.
- les moyens de pilotage sont configurés pour détecter un balayage digital de l'utilisateur sur la surface de la zone tactile sur une distance inférieure à 10 cm, de préférence entre 1 et 10 cm, de préférence encore entre 2 et 9 cm, par exemple entre 3 et 8 cm.
- le déplacement, i.e. un glissement, du doigt de l'utilisateur sur, i.e. au contact de, ladite zone tactile selon le premier ou le deuxième sens se fait sur une distance d'au moins 1 cm, de préférence d'au moins 2 cm, de préférence d'au moins 2.5 cm à 3 cm.
- le déplacement, i.e. un glissement, du doigt de l'utilisateur sur, i.e. au contact de, ladite zone tactile selon le premier ou le deuxième sens se fait sur une distance d'au plus 10 cm, de préférence d'au plus 9 cm, préférentiellement d'au plus 8.5 cm.
- le déplacement, i.e. un glissement, du doigt de l'utilisateur sur, i.e. au contact de, ladite zone tactile selon le premier ou le deuxième sens se fait sur une distance comprise entre 1 et 10 cm, de préférence entre 1 et 9 cm, de préférence entre 2 et 8 cm environ, typiquement de l'ordre de 3 ou 4 cm à 7 ou 8cm.
- le déplacement, i.e. un glissement, du doigt de l'utilisateur sur, i.e. au contact de, ladite zone tactile selon le premier sens se fait selon une trajectoire rectiligne ou non-rectiligne, par exemple incurvée ou autre, de préférence une trajectoire rectiligne ou approximativement rectiligne.
- le déplacement i.e. un glissement, du doigt de l'utilisateur sur, i.e. au contact de, ladite zone tactile selon le deuxième sens se fait selon une trajectoire rectiligne ou non-rectiligne, par exemple incurvée ou autre, de préférence une trajectoire rectiligne ou approximativement rectiligne.
- le premier sens de déplacement est le sens de la gauche vers la droite (i.e. sens gauche/droite) et le deuxième sens de déplacement est le sens de la droite vers la gauche (i.e. sens droite/gauche), ou inversement. On parle alors de déplacement « horizontal » (cf. Fig. 3A).
- le premier sens de déplacement est le sens haut/bas et le deuxième sens de déplacement est le sens bas/haut, ou inversement. On parle alors de déplacement « vertical » (cf. Fig. 3B).
- le premier sens de déplacement et le deuxième sens de déplacement sont des sens obliques ou inclinés, c'est-à-dire selon des directions intermédiaires entre déplacements « horizontal » et « vertical » (cf. Fig. 3C).
- il comprend par ailleurs un circuit de gaz comprenant une branche d'insufflation pour amener le gaz au patient pendant les phases d'insufflation et une branche d'exsufflation pour convoyer le gaz extrait du patient, pendant les phases d'exsufflation.
- les branches d'insufflation et d'exsufflation sont par ailleurs reliées fluidiquement à une branche commune, c'est-à-dire un tronçon de conduit commun.
- la branche commune est reliée fluidiquement au patient, via un conduit flexible et une interface respiratoire, tel que masque ou autre, par exemple un masque bucco-nasal, ou une sonde de trachéotomie ou encore un embout buccal.
- la branche d'insufflation est reliée fluidiquement à la sortie d'air de la turbine de manière à recueillir l'air délivré par la turbine, c'est-à-dire sortant de la turbine.
- les moyens de pilotage sont configurés pour opérer une phase d'insufflation comprenant une alimentation de la branche d'insufflation avec du gaz sous pression, typiquement de l'air, provenant de la sortie de ladite turbine.
- la branche d'exsufflation est reliée fluidiquement à l'entrée d'air de la turbine.
- le circuit de gaz comprend en outre une ligne d'évacuation de gaz, de préférence elle comprend une valve d'échappement.
- la ligne d'évacuation de gaz est raccordée à la branche d'insufflation, c'est-à-dire raccordée en aval de la sortie de la turbine.
- la ligne d'évacuation de gaz est en communication fluidique avec la branche d'insufflation pendant les phases d'exsufflation de manière à échapper à l'atmosphère, de préférence via la valve d'échappement, au moins une partie du gaz délivré par la turbine dans la branche d'insufflation.
- la communication fluidique entre la ligne d'évacuation de gaz et la branche d'insufflation est interrompue pendant les phases d'insufflation, par actionnement des moyens à valves agencés sur la ligne d'évacuation de gaz de sorte d'interrompre toute circulation gazeuse dans cette ligne d'évacuation de gaz.
- le circuit de gaz comprend en outre une ligne d'entrée d'air.
- la ligne d'entrée d'air est raccordée à la branche d'exsufflation, c'est-à-dire raccordée en amont de l'entrée de la turbine.
- la ligne d'entrée d'air est en communication fluidique avec la branche d'exsufflation pendant les phases d'insufflation, de manière à aspirer de l'air atmosphérique et à l'acheminer jusqu'à l'entrée de la turbine.
- la communication fluidique entre la ligne d'entrée d'air et la branche d'exsufflation est interrompue pendant les phases d'exsufflation, par actionnement des moyens à valves agencés sur la ligne d'entrée d'air, de sorte d'interrompre toute entrée d'air atmosphérique dans cette ligne d'entrée d'air.
- les circulations de gaz dans le circuit de gaz, en particulier dans la ligne d'évacuation de gaz, dans la ligne d'entrée d'air et dans les branches d'insufflation et d'exsufflation, sont contrôlées par des moyens à valves ou analogues, typiquement des valves pneumatiques, par exemple des valves à membrane.
- les moyens à valves sont commandées par des moyens de commande pneumatiques.
- les moyens de commande pneumatiques comprennent une ou des électrovannes pneumatiques, de préférence plusieurs électrovannes pneumatiques.
- les électrovannes pneumatiques sont pilotées électriquement par les moyens de pilotage.
- les moyens de pilotage commandent électriquement les électrovannes pneumatiques en fonction de la phase d'insufflation ou d'exsufflation à réaliser.
- les moyens de pilotage comprennent un contrôleur numérique.
- les moyens de pilotage comprennent au moins un microprocesseur, typiquement un microcontrôleur.
- les moyens de pilotage comprennent au moins une carte électronique comprenant au moins un microprocesseur, typiquement un microcontrôleur, mettant en œuvre au moins un algorithme.
- il comprend en outre des moyens de mémorisation, par exemple une carte mémoire de type EEPROM ou autre.
- des moyens de mémorisation sont agencés sur une carte électronique.
- la turbine est à moteur électrique.
- la turbine délivre de l'air à une pression allant jusqu'à 100 mbar.
- il comprend des moyens d'alimentation en courant électrique alimentant en courant électrique tous les composants ayant besoin de courant électrique pour fonctionner, en particulier les moyens de pilotage, la turbine motorisée, l'afficheur....
- les moyens d'alimentation électrique comprennent une (des) batterie rechargeable et/ou un cordon d'alimentation munie d'une prise de raccordement au secteur (e.g. 115/230 V AC), avec ou sans transformateur de courant.
- il comprend par ailleurs une carcasse externe rigide dans laquelle est insérée la turbine, tout ou partie du circuit de gaz, les moyens de pilotage....
- l'afficheur digital à écran tactile est aménagé sur ou incorporé dans l'une des parois de la carcasse de l'appareil.
- l'afficheur digital est du type en couleurs ou du type en noir et blanc.
- il est configuré pour opérer alternativement des phases d'insufflation et d'exsufflation successives de la turbine pendant toute la période de temps durant laquelle une pression digitale de l'utilisateur, en particulier des balayages digitaux, de la surface de la zone tactile est détectée, c'est-à-dire tant que l'utilisateur opère des « va-et-vient » ou « allers-retours » digitaux à la surface de la zone tactile en balayant en continu ladite surface de la zone tactile dans le premier puis dans le deuxième sens de déplacement, et inversement.
- les moyens de pilotage déclenchent les phases d'insufflation et d'exsufflation successives en agissant sur la turbine.
- les moyens de pilotage contrôlent la durée des phases d'insufflation et d'exsufflation successives.
- une (chaque) phase d'insufflation a une durée comprise entre 0.5 et 10 secondes, typiquement jusqu'à 5 secondes environ.
- pendant une (chaque) phase d'insufflation, la turbine est commandée pour délivrer une pression positive (P+) comprise de préférence entre 5 et 70 mbar (i.e. pression relative par rapport à la pression atmosphérique).
- une (chaque) phase d'exsufflation a une durée comprise entre 0.5 et 10 secondes, typiquement jusqu'à 5 secondes environ.
- pendant une phase (chaque) d'exsufflation, la turbine génère une pression négative ou nulle (P-) comprise de préférence entre 0 et -70 mbar (i.e. pression relative par rapport à la pression atmosphérique).
- la turbine est commandée pour atteindre une vitesse de rotation maximale de 75 000 tours/minute.
- optionnellement, la (chaque) phase d'insufflation et la (chaque) phase d'exsufflation qui la suit sont elles-mêmes suivies par une phase de pause (i.e. située entre une phase d'exsufflation et la phase d'insufflation suivante) pendant laquelle on délivre une pression de pause supérieure ou égale à 0 mbar dans la ligne d'insufflation et la ligne commune, de préférence une pression de pause entre 0 et 30 mbar, par exemple de l'ordre de 10 mbar.
- optionnellement, la phase d'insufflation et la phase d'exsufflation sont espacées l'une de l'autre par une phase transitoire permettant de préparer l'état des valves (i.e. ouverture ou fermeture complète...) et des circuits de gaz (e.g. pressions dans les lignes d'insufflation et d'exsufflation...) pour que le démarrage de la phase d'exsufflation suivante s'effectue à un débit d'exsufflation donné, préférentiellement un débit d'exsufflation maximal. Chaque phase transitoire débute en fin de phase d'insufflation, c'est-à-dire qu'elle est opérée à la fin une phase d'insufflation et avant la phase d'exsufflation suivante. Une (chaque) phase transitoire a une durée comprise entre 50 et 500 ms environ.
- l'afficheur constitue ou fait partie d'une interface homme-machine ou IHM permettant à l'utilisateur, par exemple un personnel soignant ou autre, de rentrer une ou des valeurs de consigne, notamment des valeurs de pression, de temps des différentes phases...
- il comprend une station périphérique et un module de ventilation fixés de manière détachable l'un à l'autre, la station périphérique portant l'afficheur à zone tactile, et le module de ventilation comprenant les moyens de pilotage et la turbine.
- la station périphérique vient entourer et loger le module de ventilation extractible, c'est-à-dire deux parties venant s'imbriquer de manière désolidarisable/détachable, l'une dans l'autre, la station périphérique formant « manchon » autour du module de ventilation extractible.
- le module de ventilation vient s'emboiter/s'insérer dans la station périphérique de manière extractible/détachable.
- le module de ventilation central comprend la carcasse externe rigide dans laquelle sont agencés au moins la turbine motorisée, la ligne d'insufflation, la ligne d'exsufflation, la ligne commune, les valves pneumatiques, les électrovannes pneumatiques et les moyens de pilotage...
- l'afficheur à zone tactile est agencé sur la station périphérique.
- l'afficheur à zone tactile est détachable ou non-détachable de la station périphérique.
- l'afficheur à zone tactile est par exemple celui d'une tablette tactile, type IPad ou analogue.

L'invention va maintenant être mieux comprise grâce à la description détaillée suivante, faite à titre illustratif mais non limitatif, en référence aux figures annexées parmi lesquelles :
- la Figure 1 schématise le principe de fonctionnement d'un appareil d'insufflation et d'exsufflation de gaz selon l'invention,
- la Figure 2 schématise un mode de réalisation d'un écran tactile pour l'appareil de la Figure 1,
- les Figures 3A-3C schématisent des exemples de sens de déplacement sur la zone tactile de l'appareil, et
- les Figures 4 et 5 schématisent un mode de réalisation d'un appareil selon l'invention comprenant une station périphérique et un module de ventilation extractible.

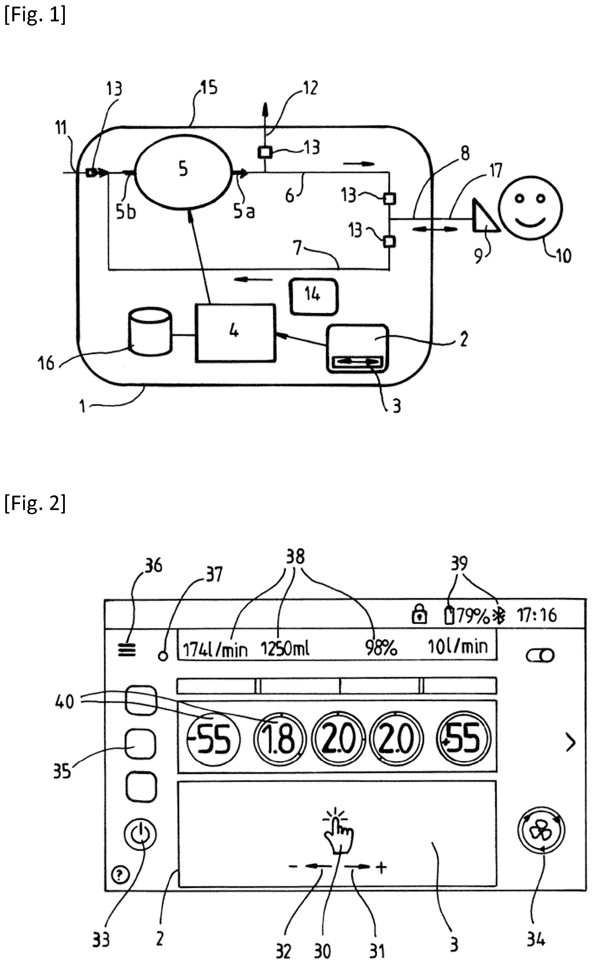

La Figure 1 schématise le principe de fonctionnement d'un appareil 1 d'insufflation et d'exsufflation de gaz selon l'invention, c'est-à-dire d'un appareil d'assistance à la toux, avantageusement portable, destiné au traitement thérapeutique de personnes, i.e. de patients, souffrant de troubles respiratoires nécessitant une aide à l'évacuation des sécrétions pulmonaires.

L'appareil 1 d'insufflation et d'exsufflation de gaz comprend un afficheur 2 digital comprenant une zone tactile 3, i.e. écran numérique de type *touch pad,* et des moyens de pilotage 4, par exemple un contrôleur numérique incorporant au moins un microprocesseur, typiquement un microcontrôleur, mettant en œuvre un ou des algorithmes servant notamment au pilotage d'une turbine motorisée 5.

Une telle turbine 5, aussi appelée micro-soufflante ou compresseur d'air, est généralement équipée d'un moteur électrique protégé par un carter et entraînant, via un arbre ou axe-moteur, une roue à ailette agencée dans une volute de sorte d'aspirer de l'air ambiant et de le redistribuer à pression et/ou à débit et/ou à volume donné(s).

Préférentiellement, un filtre, par exemple HEPA, est agencé en amont de la turbine 5 de manière à purifier l'air avant son entrée dans la turbine 5, en y éliminant tout ou partie des poussières, microorganismes ou autres aérosols pouvant s'y trouver.

La turbine 5 est conçue pour délivrer de l'air à une pression allant jusqu'à 100 mbar environ. La vitesse de rotation maximale du moteur de la turbine 5 peut atteindre 75 000 tr/min. La turbine 5 est reliée fluidiquement à un circuit de gaz 6, 7, c'est-à-dire des conduits ou passages de gaz, comprenant une branche d'insufflation 6 pour amener le gaz au patient 10, pendant les phases d'insufflation de gaz, et une branche d'exsufflation 7 pour convoyer le gaz extrait du patient 10, pendant les phases d'exsufflation, c'est-à-dire d'aspiration des sécrétions. Les branches d'insufflation 6 et d'exsufflation 7 sont par ailleurs reliées fluidiquement à une branche commune 8, c'est-à-dire un tronçon de conduit commun.

La branche d'insufflation 6 est reliée fluidiquement à la sortie 5a d'air de la turbine de 5 manière à recueillir l'air délivré par la turbine 5, c'est-à-dire sortant de la turbine 5, alors que la branche d'exsufflation 7 est reliée fluidiquement à l'entrée d'air 5b de la turbine 5.

Par ailleurs, la branche commune 8, c'est-à-dire le tronçon de conduit commun, est elle-même reliée fluidiquement au patient 10, via un tuyau flexible 17 et une interface respiratoire 9, tel que masque ou autre.

La branche d'insufflation 6 par ailleurs reliée fluidiquement à l'atmosphère via une ligne d'évacuation de gaz 12, tel un conduit ou passage de gaz, pour permettre d'envoyer vers l'atmosphère ambiant du gaz contenu dans la branche d'insufflation 6, en particulier durant les phases d'exsufflation de gaz.

Par ailleurs, la branche d'exsufflation 7 est, quant à elle, reliée fluidiquement à l'atmosphère ambiant via une ligne d'entrée d'air 11, tel un conduit ou passage de gaz, pour permettre d'alimenter la turbine 5 en air, en particulier durant les phases d'insufflation de gaz.

Les circulations de gaz dans la ligne d'évacuation de gaz 12, dans la ligne d'entrée d'air 11 et dans le circuit de gaz 6, 7, en particulier dans les branches d'insufflation 6 et d'exsufflation 7, sont contrôlées par des moyens à valves 13, telles des valves pneumatiques, pouvant elles-mêmes être commandées pneumatiquement par des électrovannes pneumatiques pilotées électriquement par les moyens de pilotage 4.

L'appareil 1 comprend aussi des moyens d'alimentation électrique 14, typiquement une (ou plusieurs) batterie rechargeable ou un cordon d'alimentation munie d'une prise de raccordement au secteur (e.g. 115/230 V AC), ou les deux, et éventuellement un transformateur de courant. Lorsque l'appareil 1 doit être portable ou portatif, on prévoit au moins une batterie rechargeable.

Les moyens d'alimentation électrique 14 alimentent les différents composants de l'appareil 1 requérant de la puissance électrique (i.e. du courant) pour fonctionner, notamment le moteur de la turbine 5, l'afficheur digital 2 à écran tactile 3, les moyens de pilotage 4....

Une carcasse externe 15 rigide permet de protéger notamment la turbine 5, tout ou partie du circuit de gaz 6, 7, les moyens de pilotages 4....

L'afficheur digital 2 à écran tactile 1 peut être aménagé sur ou incorporé dans l'une des parois de l'appareil 1, préférentiellement la paroi du dessus. L'afficheur digital 2 à écran tactile 1 peut être solidarisé de manière amovible ou non-amovible (i.e. fixe) par exemple à la carcasse 15 de l'appareil 1.

Par ailleurs, l'afficheur digital 2 peut être à affichage en couleurs, en noir et blanc ou les deux, par exemple on peut prévoir de basculer d'un affichage couleur à un affichage noir et blanc, ou inversement.

A titre d'exemple, on peut utiliser l'écran tactile d'une tablette numérique ou un écran tactile indépendant.

Selon la présente invention, il est prévu un système de contrôle manuel amélioré permettant à un utilisateur de déclencher manuellement les phases d'insufflation et d'exsufflation.

Plus précisément, selon l'invention, les moyens de pilotage 4 sont configurés pour commander la turbine 5 en réponse à la détection d'au moins une pression digitale d'un utilisateur sur la zone tactile 3 de l'afficheur 2, typiquement un pavé tactile ou analogue.

La pression digitale détectée est « dynamique », c'est-à-dire que c'est un déplacement, typiquement un glissement, du doigt de l'utilisateur sur la zone tactile qui est détecté.

Selon l'invention, lorsque l'appareil 1, i.e. les moyens de pilotage 4, détecte un contact du doigt d'un utilisateur avec la zone tactile 3 de l'afficheur 2, c'est-à-dire le fait que l'utilisateur touche la zone tactile 3 avec son doigt, typiquement avec l'index, et surtout un glissement du doigt de l'utilisateur sur la zone tactile 3, il va rétroagir sur la turbine 5 pour déclencher soit une phase d'insufflation, soit une phase d'exsufflation.

La phase à déclencher, c'est-à-dire insufflation ou exsufflation, peut être déterminée en fonction du sens de déplacement du doigt de l'utilisateur sur la zone tactile 3, c'est-à-dire du sens (i.e. mouvement) du balayage digital opéré par le doigt de l'utilisateur sur la surface de la zone tactile 3.

De préférence, un déclenchement d'une phase d'insufflation (i.e. inspiration) de gaz se produit lorsque l'utilisateur opère un glissement de son doigt sur la zone tactile 3 selon un sens conventionnel prédéfini, notamment mémorisé, par exemple de la gauche vers la droite. Un glissement de son doigt dans le sens opposé engendre alors un déclenchement d'une phase d'exsufflation (i.e. expiration).

En d'autres termes, les moyens de pilotage 4 sont avantageusement configurés pour opérer un démarrage de la turbine 5 et déclencher ensuite une phase d'insufflation de la turbine 5 en réponse à la détection d'un déplacement, i.e. un glissement du doigt de l'utilisateur sur la zone tactile 3, par exemple lorsque le doigt de l'utilisateur balaie la surface de la zone tactile 3 préférentiellement dans le sens gauche/droite, c'est-à-dire allant de la gauche vers la droite, ou selon un autre sens, c'est-à-dire en réalisant des « va-et-vient » ou « allers-retours » digitaux à la surface de la zone tactile, sur une distance comprise entre 1 et 10 cm, typiquement entre 2 et 9 cm.

Durant toute la durée du balayage, c'est-à-dire des « va-et-vient » ou « allers-retours » digitaux, le doigt de l'utilisateur se déplace en étant maintenu en contact avec la surface de la zone tactile 3.

A titre d'exemple, plusieurs sens de déplacement possibles ont été schématisés par des flèches de direction sur les Figures 3A-3C, à savoir le sens (i.e. direction):
- de la gauche vers la droite ou gauche/droite, et inversement droite/gauche, aussi appelé déplacement « horizontal », en Fig. 3A,
- du haut vers le bas ou haut/bas et inversement bas/haut, aussi appelé déplacement « vertical », en Fig. 3B, et
- des sens obliques (i.e. inclinés), selon des directions intermédiaires entre déplacements « horizontal » et « vertical », comme illustré en Fig. 3C.

Par ailleurs, la trajectoire suivie par le doigt de l'utilisateur lorsqu'il se déplace sur la zone tactile 3 de l'écran numérique 2 dans le sens choisi, peut être rectiligne, approximativement rectiligne ou non-rectiligne, par exemple légèrement incurvée. De préférence, la trajectoire suivie par le doigt de l'utilisateur est rectiligne ou approximativement rectiligne.

Durant une phase d'insufflation, la turbine 5 fournit de l'air sous pression (par exemple de l'ordre de 40 mbar) à la branche d'insufflation 6 du circuit de gaz 6, 7, qui est acheminé jusqu'au patient 10, via ladite branche d'insufflation 6, la branche commune 8 et l'interface respiratoire 9. L'air alimentant l'entrée 5a de la turbine 5 provient de l'atmosphère ambiante et y est acheminé via la ligne d'entrée d'air 11.

A l'inverse, les moyens de pilotage 4 sont configurés pour déclencher une phase d'exsufflation de la turbine 5 en réponse à la détection d'un déplacement du doigt de l'utilisateur sur la zone tactile 3 selon un deuxième sens de déplacement différent du premier sens de déplacement, i.e. préférentiellement des sens opposés, par exemple lorsque le doigt de l'utilisateur balaie la surface de la zone tactile 3 dans le sens droite/gauche, c'est-à-dire allant de la droite vers la gauche.

Durant une phase d'exsufflation, la turbine 5 aspire l'air se trouvant dans la branche d'exsufflation 7 du circuit de gaz 6, 7, c'est-à-dire qu'elle y crée une dépression (i.e. une pression négative ou vide, par exemple de l'ordre de -40 mbar), qui se propage jusqu'aux voies aériennes du patient 10, via la branche commune 8 et l'interface respiratoire 9, de sorte de l'assister dans l'évacuation de ses sécrétions pulmonaires ou autres. L'air aspiré par la turbine 5 est rejeté à l'atmosphère via la ligne d'évacuation de gaz 12.

Ainsi, en opérant des « va-et-vient » ou des « allers-retours » en balayant en continu la surface de la zone tactile 3 de gauche à droite et, inversement, l'utilisateur va commander alternativement des phases d'insufflation et d'exsufflation successives de la turbine 5, et ce, pendant toute la période de temps durant laquelle l'utilisateur opérera ces balayages de la surface de la zone tactile 3 avec son doigt, par exemple son index.

En général, l'utilisateur balaie la surface de la zone tactile sur une distance de l'ordre de quelques centimètres, par exemple de 3 à 8 cm environ, que ce soit dans un sens ou dans l'autre, c'est-à-dire par exemple gauche/droite ou droite/gauche.

On prévoit aussi des moyens de mémorisation 16, par exemple une carte mémoire de type EEPROM ou autre, permettant de mémoriser les consignes ou paramètres, tels que pression, vitesse, débit, volume, durées...., à appliquer durant les phases d'insufflation et d'exsufflation. Ces consignes ou paramètres y seront retrouvés par les moyens de pilotage 4 qui sont reliés électriquement aux moyens de mémorisation 16, par exemple en étant agencés sur une même carte électronique ou sur des cartes électroniques reliées électriquement entre elles.

En d'autres termes, selon la présente invention, il est proposé d'opérer une commande de contrôle de l'appareil 1 d'assistance de toux via une action de l'utilisateur réalisée sur la zone tactile 3 de l'afficheur 2, tel un pavé tactile ou analogue, c'est-à-dire un balayage/glissement du doigt sur la surface de cette zone tactile 3 de la gauche vers la droite et inversement.

Pour déclencher une phase d'insufflation, il suffit de poser un doigt, tel l'index, sur la partie commande du pavé tactile, c'est-à-dire la zone tactile, et d'exercer un mouvement dans un premier sens, en particulier en fonction d'une convention choisie, par exemple gauche/droite ou autre. Ce mouvement est alors détecté et interprété par un algorithme mis en œuvre par les moyens de pilotage 4, notamment un microcontrôleur, comme étant une commande d'insufflation et l'information est transmise au contrôleur numérique 4 de l'appareil 1 qui va alors commander la turbine 5 pour la démarrer ainsi que l'ouverture ou la fermeture des moyens à valves 13, et ainsi fournir de l'air au patient 10, via la ligne d'insufflation 6 et la ligne commune 8, et ce, en respectant les consignes de thérapie préalablement configurées sur l'appareil.

Ensuite, pour déclencher une phase d'exsufflation immédiatement après la phase d'insufflation, il suffit que l'utilisateur continue à toucher la zone tactile 3 mais en changeant le sens du mouvement de balayage qu'il opère avec son doigt, c'est-à-dire qu'il opère un glissement de son doigt dans un deuxième sens, par exemple le sens droite/gauche ou autre.

Ce mouvement inverse est alors détecté et interprété comme étant une commande d'exsufflation, comme précédemment, et l'information est, là encore transmise au contrôleur numérique 4 de l'appareil 1 qui va alors commander la turbine 5 ainsi que l'ouverture ou la fermeture des moyens à valves 13, et ainsi extraire du gaz des voies aériennes supérieures du patient 10 grâce à une mise en dépression (vide partiel) de la ligne d'exsufflation de gaz 7 et la ligne commune 8, et ce, là encore, en respectant les consignes de thérapie préalablement configurées au niveau de l'appareil 1.

Dit autrement, si le doigt exerce un mouvement dans le sens opposé à celui commandant l'insufflation, une commande d'exsufflation est transmise au générateur d'air, c'est-à-dire la turbine 5, qui opère instantanément une dépression dans la ligne d'exsufflation 7, la ligne commune 8 et donc aussi dans le tuyau flexible 17 reliant l'appareil 1 au patient 10.

Dès lors, tant que l'utilisateur va opérer des glissements de son doigt sur la surface externe de la zone tactile 3, c'est-à-dire des mouvements de « va-et-vient » ou des « aller-retour », alternativement, dans le premier et le deuxième sens de déplacement, ces mouvements seront détectés et l'appareil 1 sera alors être commandé par les moyens de pilotage 5 pour déclencher successivement et alternativement des phases d'insufflation et d'exsufflation.

D'une façon générale, selon l'invention, la détection est réalisée en fonction de :
- le pression du doigt sur le pavé tactile indiquant la présence d'un opérateur qui souhaite établir une commande, et
- le déplacement (en pixel) associé à (l'angle de) la trajectoire du mouvement du doigt qui traduit la nature de l'action à mener, à savoir une insufflation ou une exsufflation de gaz.

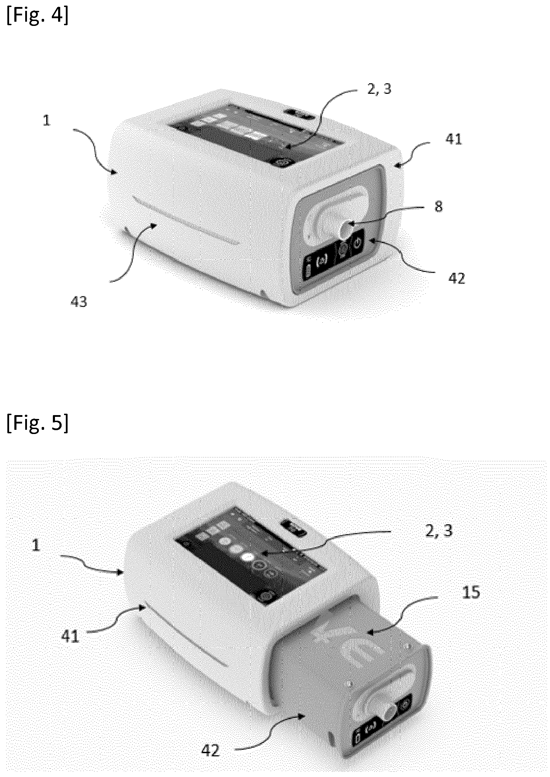

La Figure 2 schématise un mode de réalisation d'un écran ou afficheur digital 2 à zone tactile 3 équipant un appareil 1 d'insufflation et d'exsufflation de gaz selon l'invention, c'est-à-dire d'un appareil d'assistance à la toux, par exemple celui des Figures 3 et 4.

Comme on le voit, dans ce mode de réalisation, la zone tactile 3 comprend une représentation graphique de main avec index déployé pour rappeler à l'utilisateur qu'il s'agit d'une zone tactile, ainsi des flèches 31, 32 pointant dans des directions opposées pour lui indiquer les sens de balayage digital de la zone tactile, et des signes « + » et « - » pour lui indiquer l'insufflation et l'exsufflation, respectivement.

Par ailleurs, l'afficheur numérique 2 comprend ici d'autres touches numériques activables par pression digitale de l'utilisateur, notamment des touches de mise sous tension/extinction de l'appareil (off) 33 et de démarrage/arrêt 34 de la ventilation et/ou du traitement du patient (start/stop), plusieurs touches programmables 35 (Prog1, Prog2, Prog3) 35, une touche d'accès au menu de l'appareil 36 (Menu), une touche 37 d'accès aux réglages (Settings)....

En outre, l'afficheur numérique 2 affiche différentes informations utiles comme :
- des mesures 38 relatives aux flux gazeux : débit de pic (peak flow), volume courant de gaz (tidal volume), saturation en oxygène (SpO₂), débit de fuite (leak),
- des informations 39 relatives au fonctionnement de l'appareil 1, tel que niveau de charge de la batterie rechargeable, l'établissement d'une communication Bluetooth, le verrouillage de certaines fonctions ou touches (symbole cadena)...
- des valeurs 40 de niveaux de pression et de durées pour l'insufflation et l'exsufflation, ou encore le temps de pause.

Bien entendu, l'écran d'affichage 2 peut se présenter sous une configuration différente et/ou afficher d'autres informations et/ou comprendre d'autres touches de fonction ou autres indications.

Les Figures 4 et 5 représentent des vues générales d'un mode de réalisation d'un appareil d'insufflation et d'exsufflation de gaz 1 selon l'invention, aussi appelé appareil d'assistance à la toux, utilisable pour traiter des personnes souffrant de troubles respiratoires nécessitant une aide à l'évacuation des sécrétions pulmonaires.

Cet appareil 1 fonctionne comme exposé ci-avant, notamment selon le principe de fonctionnement illustré en Figure 1, et incorpore un afficheur 2 tel que représenté en Figure 2.

Dans ce mode de réalisation, l'appareil 1 comprend deux parties 41, 42 venant s'imbriquer l'une dans l'autre et pouvant être séparées/détachées l'une de l'autre, à savoir une station périphérique 41 venant entourer et abriter un module de ventilation 42 central extractible.

Plus précisément, le module de ventilation 42 est extractible/détachable. Il vient s'emboiter/s'insérer ou, à l'inverse, peut être retiré/extrait de la station périphérique 41 selon un mouvement de translation, comme montré en Figure 5 où le module de ventilation 42 est à moitié inséré dans la station périphérique 41.

Le module de ventilation 42 extractible comprend la turbine 5, les lignes d'insufflation 6, d'exsufflation 7 et commune 8, les moyens de pilotage 4, etc... lesquels sont protégés par la carcasse rigide 15 entourant et délimitant ce module 42.

Dans ce cas, l'afficheur digital 2 à écran tactile 1 n'est pas porté par l'une des parois de la carcasse 15 du module de ventilation 42 de l'appareil 1 mais est fixé à la station périphérique 41. L'afficheur digital 2 à écran tactile 1 peut y être solidarisé de manière amovible ou non-amovible (i.e. fixe).

En fait, l'afficheur digital 2 à écran tactile 1 constitue ici une interface homme-machine ou IHM portée par la coque rigide 43 de la station périphérique 41, par exemple une coque en polymère ou analogue. L'IHM permet à l'utilisateur de rentrer des valeurs de consigne dans l'appareil 1, notamment des valeurs de pression haute et basse, des durées... et à afficher des informations variées, comme expliqué ci-avant (cf. Fig. 2).

Il est aussi prévu des moyens d'alimentation en courant électrique alimentant en courant électrique les différents composants de l'appareil 1 nécessitant de l'être, à savoir notamment la turbine 1 motorisée, les moyens de pilotage 4, l'afficheur 2.... ou d'autres composants. Les moyens d'alimentation en courant électrique comprennent préférentiellement une (ou plusieurs) batterie ou pile, de préférence rechargeable, et une prise électrique et un cordon électrique de raccordement au secteur, e.g. 115/230 V AC, avec ou sans transformateur de courant.

Dans le mode de réalisation des Figures 4 et 5, l'appareil 1 comprend deux modules 41, 42 venant s'insérer l'un dans l'autre ; toutefois, il est entendu que l'appareil 1 selon l'invention pourrait ne comprendre qu'un seul module intégrant l'ensemble des composants de l'appareil 1.

D'une manière générale, un appareil d'insufflation et d'exsufflation de gaz, aussi appelé appareil à toux, selon la présente invention peut être utilisé chez des patients incapables de gérer seuls leurs sécrétions de sorte de leur fournir des insufflations et exsufflations de gaz (i.e. d'air), que ces patients soient des patients adultes ou pédiatriques. Il peut être utilisé à domicile ou à l'hôpital, avec des interfaces respiratoires invasives (e.g. sondes trachéales) ou non-invasives (e.g. masques).

L'appareil d'insufflation et d'exsufflation de gaz selon la présente invention présente notamment l'avantage de pouvoir réagir immédiatement, c'est-à-dire sans délai de latence, en fonction des glissements digitaux de « va-et-vient » opérés par l'utilisateur sur la surface sensible de la zone tactile de l'afficheur.

## Revendications

1. Appareil (1) d'insufflation et d'exsufflation de gaz comprenant un afficheur (2) comprenant une zone tactile (3), et des moyens de pilotage (4) commandant une turbine motorisée (5), les moyens de pilotage (4) étant configurés pour commander la turbine (5) en réponse à la détection d'au moins une pression digitale d'un utilisateur sur la zone tactile (3) de l'afficheur (2), **caractérisé en ce que** les moyens de pilotage (4) sont configurés pour :
a) opérer un démarrage de la turbine (5) et déclencher une phase d'insufflation de la turbine (5) en réponse à la détection d'un déplacement du doigt de l'utilisateur sur ladite zone tactile (3) selon un premier sens de déplacement, et
b) déclencher une phase d'exsufflation de la turbine (5) en réponse à la détection d'un déplacement du doigt de l'utilisateur sur ladite zone tactile (3) selon un deuxième sens de déplacement, le premier sens de déplacement et le deuxième sens de déplacement étant des sens opposés.

2. Appareil selon la revendication précédente, **caractérisé en ce que** les moyens de pilotage (4) sont configurés pour détecter un balayage digital de l'utilisateur sur la surface de la zone tactile (3) sur une distance inférieure à 10 cm, de préférence entre 2 et 9 cm.

3. Appareil selon la revendication 2, **caractérisé en ce que** la distance de balayage digital sur la surface de la zone tactile (3) est comprise entre 3 et 8 cm.

4. Appareil selon l'une des revendications précédentes, **caractérisé en ce que** les moyens de pilotage (4) sont configurés pour opérer une phase d'insufflation de la turbine (5) comprenant une fourniture de gaz par ladite turbine (5).

5. Appareil selon l'une des revendications précédentes, **caractérisé en ce qu'**il est configuré pour opérer alternativement des phases d'insufflation et d'exsufflation successives de la turbine (5) pendant toute la période de temps durant laquelle une pression digitale de l'utilisateurs, de la surface de la zone tactile (3) est détectée, en particulier un balayage digital.

6. Appareil selon l'une des revendications précédentes, **caractérisé en ce que** le premier sens de déplacement et le deuxième sens de déplacement sont des sens prédéfinis, de préférence modifiables

7. Appareil selon l'une des revendications précédentes, **caractérisé en ce que** le premier sens de déplacement est le sens de la gauche vers la droite et le deuxième sens de déplacement est le sens de la droite vers la gauche, ou inversement.

8. Appareil selon l'une des revendications 1 à 5, **caractérisé en ce que** le premier sens de déplacement correspond à la direction du premier déplacement du doigt de l'utilisateur sur la zone tactile (3).

9. Appareil selon la revendication 8, **caractérisé en ce que** les moyens de pilotage (4) sont configurés pour déclencher une phase d'insufflation de gaz par la turbine (5), lors du premier glissement du doigt de l'utilisateur sur la zone tactile (3).

10. Appareil selon la revendication 8 ou 9, **caractérisé en ce que** les moyens de pilotage (4) sont configurés pour mémoriser le sens du premier glissement du doigt de l'utilisateur sur la zone tactile (3) en tant que premier sens de déplacement.

11. Appareil selon l'une des revendications précédentes, **caractérisé en ce que** :
- chaque phase d'insufflation de la turbine (5) a une durée comprise entre 0.5 et 10 secondes et/ou
- chaque phase d'exsufflation de la turbine (5) a une durée comprise entre 0.5 et 10 secondes.

12. Appareil selon l'une des revendications précédentes, **caractérisé en ce que** :
- pendant chaque phase d'insufflation, la turbine (5) est commandée pour délivrer une pression positive (P+) comprise entre 5 et 70 mbar, et/ou
- pendant chaque phase d'exsufflation, la turbine (5) génère une pression négative ou nulle (P-) comprise de préférence entre 0 et -70 mbar.

13. Appareil selon l'une des revendications précédentes, **caractérisé en ce qu'**il comprend une station périphérique (41) et un module de ventilation (42) fixés de manière détachable l'un à l'autre, la station périphérique (41) portant l'afficheur (2) à zone tactile (3), et le module de ventilation (42) comprenant les moyens de pilotage (4) et la turbine (5).

14. Appareil selon l'une des revendications précédentes, **caractérisé en ce que** les moyens de pilotage (4) comprennent au moins un microprocesseur, typiquement un microcontrôleur, mettant en œuvre au moins un algorithme.

15. Appareil selon l'une des revendications précédentes, **caractérisé en ce qu'**il comprend en outre des moyens de mémorisation, par exemple une carte mémoire.

## Patentansprüche

1. Einrichtung (1) zum Einblasen und Ausblasen von Gas, umfassend ein Anzeigegerät (2), das eine Berührungsfläche (3) umfasst, und Steuermittel (4), die eine motorisierte Turbine (5) steuern, wobei die Steuermittel (4) zum Betätigen der Turbine (5) als Reaktion auf die Detektion von mindestens einem digitalen Drücken eines Anwenders auf die Berührungsfläche (3) des Anzeigegeräts (2) konfiguriert sind, **dadurch gekennzeichnet, dass** die Steuermittel (4) konfiguriert sind zum:
a) Durchführen eines Starts der Turbine (5) und Auslösen einer Einblasphase der Turbine (5) als Reaktion auf die Detektion einer Bewegung des Fingers des Anwenders auf der Berührungsfläche (3) gemäß einer ersten Bewegungsrichtung, und
b) Auslösen einer Ausblasphase der Turbine (5) als Reaktion auf die Detektion einer Bewegung des Fingers des Anwenders auf der Berührungsfläche (3) gemäß einer zweiten Bewegungsrichtung, wobei die erste Bewegungsrichtung und die zweite Bewegungsrichtung entgegengesetzte Richtungen sind.

2. Einrichtung nach dem vorstehenden Anspruch, **dadurch gekennzeichnet, dass** die Steuermittel (4) konfiguriert sind zum Detektieren eines digitalen Überstreichens des Anwenders auf der Oberfläche der Berührungsfläche (3) über eine Strecke kürzer als 10 cm, vorzugsweise zwischen 2 und 9 cm.

3. Einrichtung nach Anspruch 2, **dadurch gekennzeichnet, dass** die Strecke der digitalen Überstreichung auf der Oberfläche der Berührungsfläche (3) im Bereich zwischen 3 und 8 cm liegt.

4. Einrichtung nach einem der vorstehenden Ansprüche, **dadurch gekennzeichnet, dass** die Steuermittel (4) konfiguriert sind zum Durchführen einer Einblasphase der Turbine (5), umfassend ein Bereitstellen von Gas durch die Turbine (5).

5. Einrichtung nach einem der vorstehenden Ansprüche, **dadurch gekennzeichnet, dass** sie konfiguriert ist zum abwechselnden Durchführen der aufeinanderfolgenden Einblas- und Ausblasphasen der Turbine (5) während des gesamten Zeitraums, im Laufe dessen ein digitales Drücken des Anwenders, der Oberfläche der Berührungsfläche (3), insbesondere eine digitale Überstreichung detektiert wird.

6. Einrichtung nach einem der vorstehenden Ansprüche, **dadurch gekennzeichnet, dass** die erste Bewegungsrichtung und die zweite Bewegungsrichtung vordefinierte, vorzugsweise modifizierbare Richtungen sind.

7. Einrichtung nach einem der vorstehenden Ansprüche, **dadurch gekennzeichnet, dass** die erste Bewegungsrichtung die Richtung von links nach rechts ist und die zweite Bewegungsrichtung die Richtung von rechts nach links ist, oder umgekehrt.

8. Einrichtung nach einem der Ansprüche 1 bis 5, **dadurch gekennzeichnet, dass** die erste Bewegungsrichtung der Richtung der ersten Bewegung des Fingers des Anwenders auf der Berührungsfläche (3) entspricht.

9. Einrichtung nach Anspruch 8, **dadurch gekennzeichnet, dass** die Steuermittel (4) konfiguriert sind, um beim ersten Gleiten des Fingers des Anwenders auf der Berührungsfläche (3) eine Einblasphase von Gas durch die Turbine (5) auszulösen.

10. Einrichtung nach Anspruch 8 oder 9, **dadurch gekennzeichnet, dass** die Steuermittel (4) zum Speichern der Richtung des ersten Gleitens des Fingers des Anwenders auf der Berührungsfläche (3) als erste Bewegungsrichtung konfiguriert sind.

11. Einrichtung nach einem der vorstehenden Ansprüche, **dadurch gekennzeichnet, dass**:
- jede Einblasphase der Turbine (5) eine Dauer im Bereich zwischen 0,5 und 10 Sekunden aufweist und/oder
- jede Ausblasphase der Turbine (5) eine Dauer im Bereich zwischen 0,5 und 10 Sekunden aufweist.

12. Einrichtung nach einem der vorstehenden Ansprüche, **dadurch gekennzeichnet, dass**:
- während jeder Einblasphase die Turbine (5) zum Liefern eines Überdrucks (P+) im Bereich zwischen 5 und 70 mbar gesteuert wird, und/oder
- während jeder Ausblasphase die Turbine einen Unter- oder Nulldruck (P-), vorzugsweise im Bereich zwischen 0 und -70 mbar, erzeugt.

13. Einrichtung nach einem der vorstehenden Ansprüche, **dadurch gekennzeichnet, dass** sie eine Außenstation (41) und ein Belüftungsmodul (42) umfasst, die auf lösbare Weise aneinander befestigt sind, wobei die Außenstation (41) das Anzeigegerät (2) mit Berührungsfläche (3) trägt und das Belüftungsmodul (42) die Steuermittel (4) und die Turbine (5) umfasst.

14. Einrichtung nach einem der vorstehenden Ansprüche, **dadurch gekennzeichnet, dass** die Steuermittel (4) mindestens einen Mikroprozessor, üblicherweise einen Mikrocontroller, umfassen, der mindestens einen Algorithmus implementiert.

15. Einrichtung nach einem der vorstehenden Ansprüche, **dadurch gekennzeichnet, dass** sie weiter Speichermittel umfasst, zum Beispiel eine Speicherkarte.

## Claims

1. Device (1) for insufflation and exsufflation of gas comprising a display unit (2) comprising a tactile zone (3), and control means (4) controlling a motorised turbine (5), the control means (4) being configured to control the turbine (5) in response to the detection of at least one finger pressure of a user on the tactile zone (3) of the display unit (2), **characterised in that** the control means (4) are configured to:
a) perform a start-up of the turbine (5) and trigger an insufflation phase of the turbine (5) in response to the detection of a displacement of the finger of the user on said tactile zone (3) along a first direction of displacement, and
b) trigger an exsufflation phase of the turbine (5) in response to the detection of a displacement of the finger of the user on said tactile zone (3) along a second direction of displacement, the first direction of displacement and the second direction of displacement being opposite directions.

2. Device according to the preceding claim, **characterised in that** the control means (4) are configured to detect a finger scanning of the user over the surface of the tactile zone (3) over a distance less than 10 cm, preferably between 2 and 9 cm.

3. Device according to claim 2, **characterised in that** the finger scanning distance over the surface of the tactile zone (3) is comprised between 3 and 8 cm.

4. Device according to one of the preceding claims, **characterised in that** the control means (4) are configured to perform an insufflation phase of the turbine (5) comprising a supplying of gas by said turbine (5).

5. Device according to one of the preceding claims, **characterised in that** it is configured to perform alternatively successive insufflation and exsufflation phases of the turbine (5) for the whole time period during which a finger pressure of the users, of the surface of the tactile zone (3) is detected, in particular a finger scanning.

6. Device according to one of the preceding claims, **characterised in that** the first direction of displacement and the second direction of displacement are predefined directions, preferably modifiable.

7. Device according to one of the preceding claims, **characterised in that** the first direction of displacement is the direction from left to right and the second direction of displacement is the direction from right to left, or vice versa.

8. Device according to one of claims 1 to 5, **characterised in that** the first direction of displacement corresponds to the direction of the first displacement of the finger of the user over the tactile zone (3).

9. Device according to claim 8, **characterised in that** the control means (4) are configured to trigger a gas insufflation phase through the turbine (5), during the first sliding of the finger of the user over the tactile zone (3).

10. Device according to claim 8 or 9, **characterised in that** the control means (4) are configured to memorise the direction of the first sliding of the finger of the user over the tactile zone (3) as first direction of displacement.

11. Device according to one of the preceding claims, **characterised in that**:
- each insufflation phase of the turbine (5) has a duration comprised between 0.5 and 10 seconds, and/or
- each exsufflation phase of the turbine (5) has a duration comprised between 0.5 and 10 seconds.

12. Device according to one of the preceding claims, **characterised in that**:
- during each insufflation phase, the turbine (5) is controlled to deliver a positive pressure (P+) comprised between 5 and 70 mbar, and/or
- during each exsufflation phase, the turbine (5) generates a negative or zero pressure (P-) comprised preferably between 0 and -70 mbar.

13. Device according to one of the preceding claims, **characterised in that** it comprises a peripheral station (41) and a ventilation module (42) detachably fixed to one another, the peripheral station (41) carrying the display unit (2) with a tactile zone (3), and the ventilation module (42) comprising the control means (4) and the turbine (5).

14. Device according to one of the preceding claims, **characterised in that** the control means (4) comprise at least one microprocessor, typically a microcontroller, implementing at least one algorithm.

15. Device according to one of the preceding claims, **characterised in that** it further comprises memorisation means, for example a memory card.
